# EUROPEAN PATENT SPECIFICATION

(11) **EP 3 125 782 B1**
(45) Date of publication and mention of the grant of the patent: **01.09.2021**
(21) Application number: 15773018.5
(22) Date of filing: 30.03.2015
(51) Int. Cl.: A61B 17/12, A61B 5/08, A61B 5/107, A61B 1/012

(54) **ANCHORING MECHANISMS AND SYSTEMS FOR ENDOLUMINAL DEVICES**
VERANKERUNGSMECHANISMUS UND -SYSTEME FÜR ENDOLUMINALE VORRICHTUNGEN
MÉCANISMES ET SYSTÈMES D'ANCRAGE POUR DISPOSITIFS ENDOLUMINAUX

(30) Priority: 31.03.2014 US 201461973142 P; 31.03.2014 US 201461973105 P; 31.03.2014 US 201461973110 P; 31.03.2014 US 201461973169 P; 31.03.2014 US 201461973137 P
(43) Date of publication of application: 08.02.2017
(73) Proprietor: Gyrus ACMI, Inc. d/b/a Olympus Surgical Technologies America, Southborough, MA 01772 (US)
(72) Inventor: DILLARD, David, H., Redmond, WA 98052 (US); JOHNSON, Timothy, J., Kent, WA 98032 (US); SHUMAN, Brandon, James, Redmond, WA 98052 (US); BAILLARGEON, Jean-Martin, Redmond, WA 98052 (US); LAUER, Lisa, Redmond, WA 98052 (US)
(74) Representative: Noack, Andreas
(86) International application number: PCT/US2015/023421
(87) International publication number: WO 2015/153507

(56) References cited:
- EP-A1- 2 475 328
- WO-A1-2013/109398
- WO-A2-2007/134587
- US-A1- 2004 143 282
- US-A1- 2012 184 985
- US-A1- 2012 209 308
- US-A1- 2012 209 308
- None

## Description

### REFERENCE TO RELATED APPLICATIONS

This application claims priority to U.S. Provisional Patent Application No. 61 /973142, filed March 31 , 2014 (Arty. Ref. No. SPIRTN.106P).

### BACKGROUND

### Field

This disclosure relates to anchoring mechanisms for devices used inside lung airways.

### Related Art

Struts having anchors are typically used to anchor devices used inside airways to lungs. Patent Application WO2007/134587 discloses a stent having flexible anchoring elements designed to pierce a lumen wall. Patent Applications US2012/0209308 and US2004/0143282 both disclose endoluminal devices having anchoring elements radially extending from a central hub. Patent Application WO2011/031981 discloses an anchoring element for endoluminal devices comprising a circumferential structure with attached protrusions. However, provided herein are devices and methods that can help anchor devices used inside airways to lungs using springs, for example.

### SUMMARY

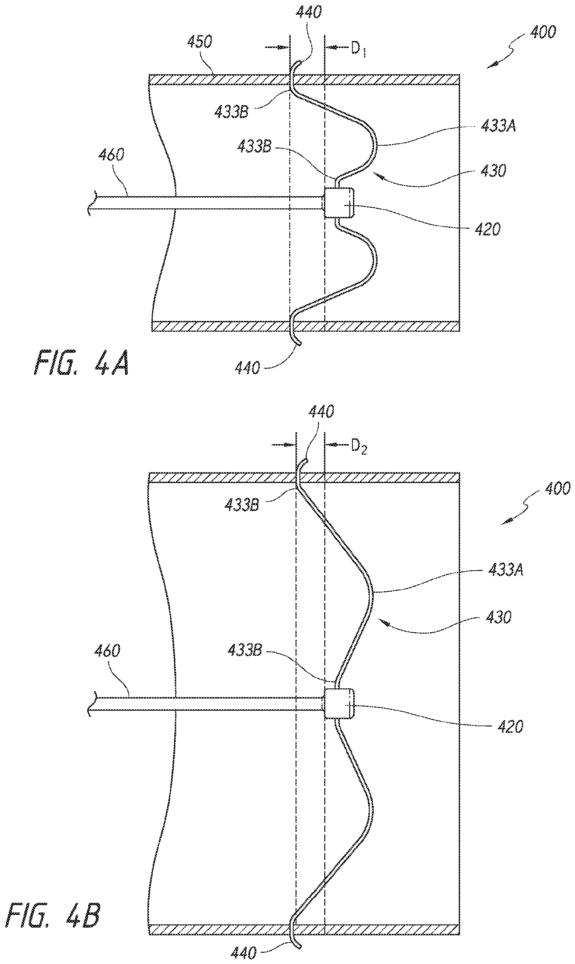

The present technology relates to anchoring mechanisms and systems for endoluminal devices. This disclosure provides a mechanism for anchoring endoluminal devices to a lumen according to claim 1. Embodiments of the invention are disclosed in figures 4 to 6 and corresponding passages of the description.

A mechanism for anchoring endoluminal devices to a lumen can comprise a hub member. The hub member can be configured to be located at a predetermined location inside the lumen. The mechanism can comprise a plurality of anchoring struts. The plurality of anchoring struts can comprise a spring portion. The spring portion can be attached to the hub member. The spring portion can be configured to extend or contract during expansion and contraction of the lumen. The mechanism can comprise an anchoring portion. The anchoring portion can connect the spring portion to the lumen. The anchoring portion can be configured to penetrate a portion of the lumen. The hub member can be positioned on a plane with the anchoring portions of the plurality of anchoring struts.

The anchoring portion can comprise a bi-directional anchoring tip. The bi-directional anchoring tip can comprise a first tip pointed to a first direction. The bi-directional anchoring tip can comprise a second tip pointed to a second direction at an angle from the first direction. At least a portion of the first tip can penetrate the lumen in a transverse direction to the lumen.

The plurality of anchoring struts can comprise equal lengths.

Each of the spring portions of the plurality of anchoring struts can extend radially outward from the hub member. Each of the spring portion of the plurality of anchoring struts can comprise a helically coiled spring. Each of the spring portion of the plurality of anchoring struts can comprise bent strut members configured to straighten during expansion of the lumen and bend during contraction of the lumen.

The bent strut members can comprise a vortex shape when viewed along a longitudinal axis of the lumen. The bent strut members can comprise a spiral shape when viewed along a longitudinal axis of the lumen. Each of the bent strut members can comprise undulating strut members having a substantially straight shape when viewed in a transverse direction.

The bent strut members can comprise one or more thin portions and one or more thick portions thicker than the thin portions. The hub member can comprise a first hub and a second hub collinear and connected to the first hub. The plurality of anchoring struts can be connected to the first hub.

An endoluminal device can comprise an operative portion. The operative portion can be configured to obstruct fluid flow inside the lumen. The endoluminal device can comprise a hub member. The hub member can be connected to the operative portion. The hub member can comprise a hub and a centering rod. The endoluminal device can comprise a plurality of anchoring struts. The plurality of anchoring struts can comprise a strut portion. The strut portion can be attached to the hub member. The strut portion can be configured to expand and contract in response to expansion and contraction of the lumen. The anchoring portion can connect the deflectable portion to the lumen. The hub member can substantially remain on or near an axial line of the lumen during movement of the lumen.

The strut portion can be connected to a sliding hub configured to slide along a length of the centering rod. The plurality of anchoring struts can comprise a plurality of quadrilateral-shaped members. Each of the quadrilateral-shaped members cars lie on a separate plane parallel to a longitudinal axis of the device.

A mechanism for anchoring endoluminal devices to a lumen can comprise a hub member. A plurality of distal anchoring struts can be connected to the hub member. The plurality of distal anchoring struts can extend radially and distally from the hub member and bend in a proximal direction. Each of the plurality of distal anchoring struts can comprise anchoring tips. The mechanism for anchoring endoluminal devices can comprise a plurality of opposing anchor struts. The plurality of opposing anchoring struts can be connected to the hub member. The plurality of opposing anchoring struts can extend proximally and radially from the hub member and bend in a distal direction. Each of the plurality of opposing anchor struts can comprise opposing anchor tips. The distal anchoring struts and the opposing anchoring struts can be configured to pinch a portion of the lumen.

A mechanism for anchoring endoluminal devices to a lumen can comprise a hub member. A plurality of anchoring struts can extend radially outward and distally from the hub member. Each of the plurality of anchoring struts can be configured to transition from a straightened configuration to a curved configuration when deployed in the lumen. Each of the plurality of anchoring struts can have a length when in the straightened configuration. The length of one or more of the plurality of anchor struts in the straightened configuration can be at least 1.5 times the fully expanded diameter of the lumen.

A mechanism for anchoring endoluminal devices to a lumen can comprise an operative portion. The operative portion can comprise a plurality of frame struts. Each frame strut can comprise a frame strut tip. A hub member can be connected to a distal end of the operative portion. A plurality of distal anchoring struts can extend radially from the hub member and bend in a proximal direction. Each distal anchoring strut can comprise an anchoring tip. The anchor tips can be positioned between the hub member and the frame strut tips. The frame strut tips and the anchoring tips can be interposed when viewed in a transverse direction.

### BRIEF DESCRIPTION OF THE DRAWINGS

The following drawings and the associated descriptions are provided to illustrate the present disclosure and do not limit the scope of the claims.
FIG. 1 shows a schematic drawing of an example of a spring-type anchoring mechanism.
FIG. 2 shows a schematic drawing of an example of a spiral-type anchoring mechanism.
FIGS. 3A and 3B show perspective views of an example of a vortex- type anchoring mechanism.
FIGS. 4A and 4B show a schematic drawing of an example of a wave- type anchoring mechanism.
FIGS. 5A and 5B show a schematic drawing of an example of a wave- type anchoring mechanism having bends near the hub.
FIG. 6 shows a perspective view an example of a wave-type anchoring mechanism with proximal struts.
FIGS. 7A and 7B show schematic drawings of examples of anchoring mechanisms having diamond-shaped anchoring struts.
FIGS. 8A and 8B show schematic drawings of an example anchoring mechanism having bowtie-shaped anchoring struts.
FIGS. 9A and 9B show perspective views of an example of a pinch-type anchoring mechanism.
FIG. 10 shows a schematic drawing of an example of an elongated anchoring strut mechanism.

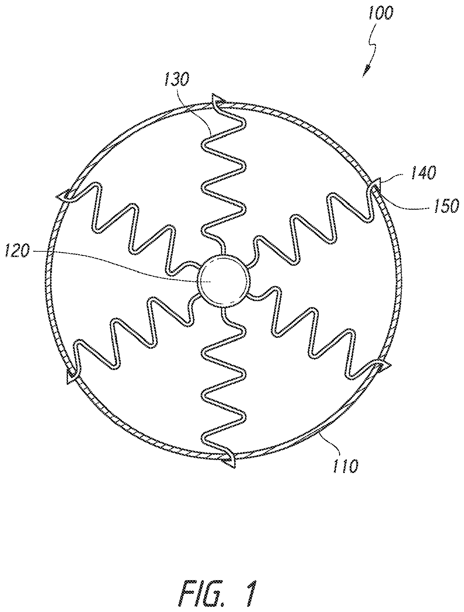

FIG. 1 1 shows an example shape of a pinch-type anchoring mechanism having elongated distal anchors.

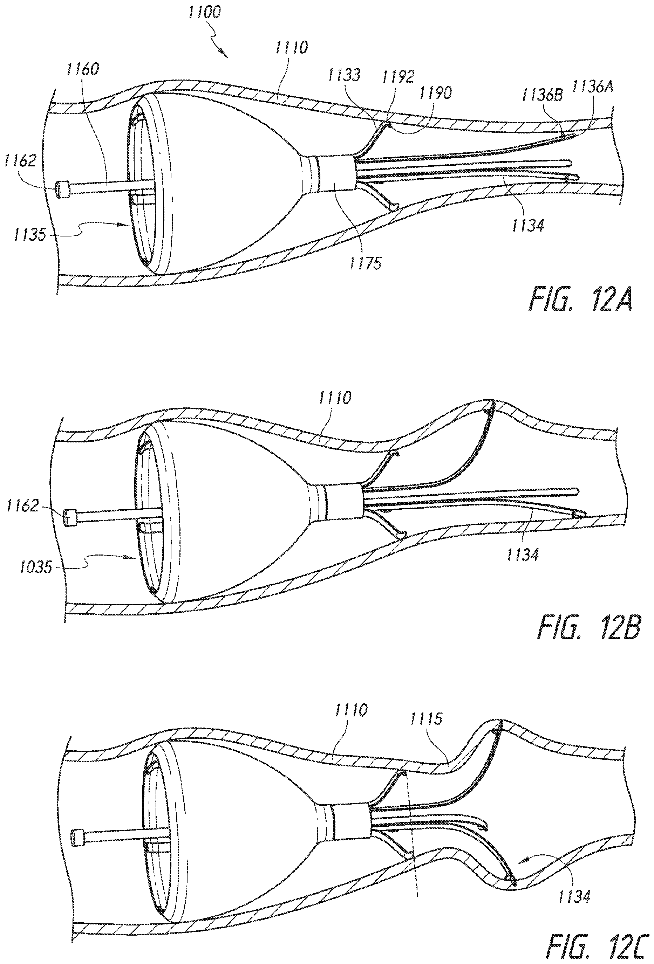

FIGS. 12A to 12C show an example of a pinch-type anchoring mechanism having elongated distal anchors with an operative portion.

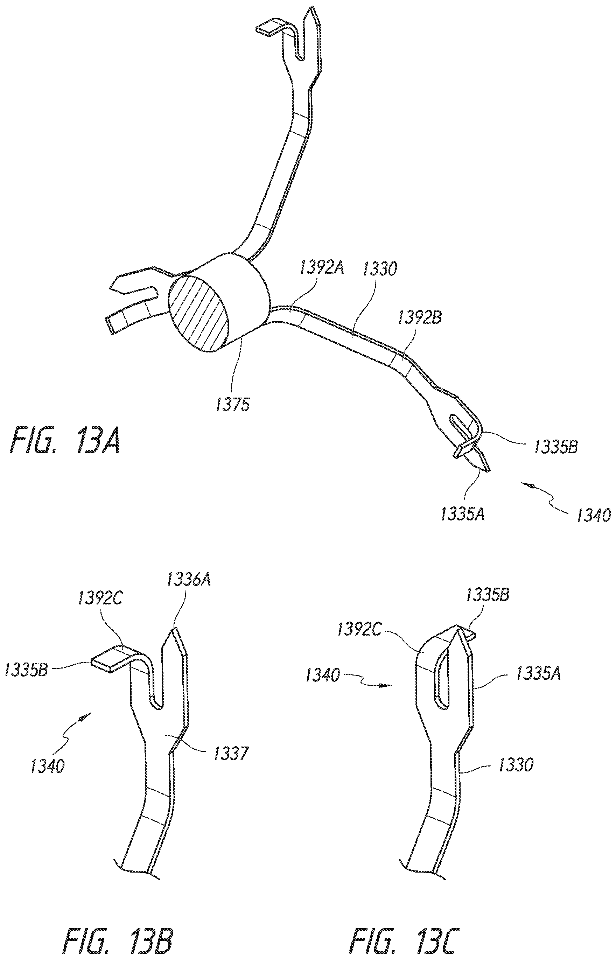

FIGS. 13A to 13C show different views of an example of a bidirectional anchoring tip.

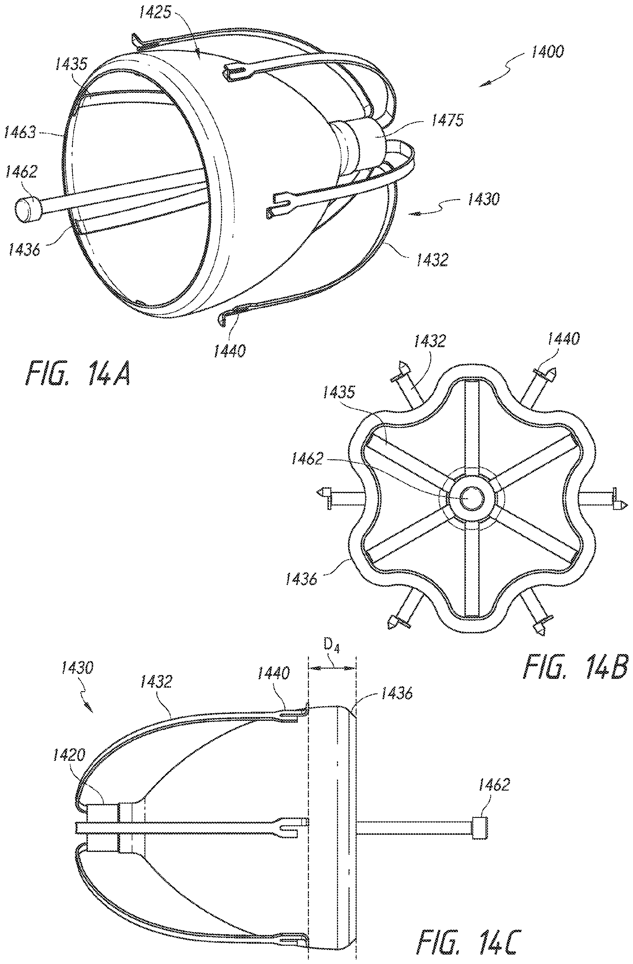

FIG. 14A to 14C show an example of an anchoring mechanism having interposing anchors.

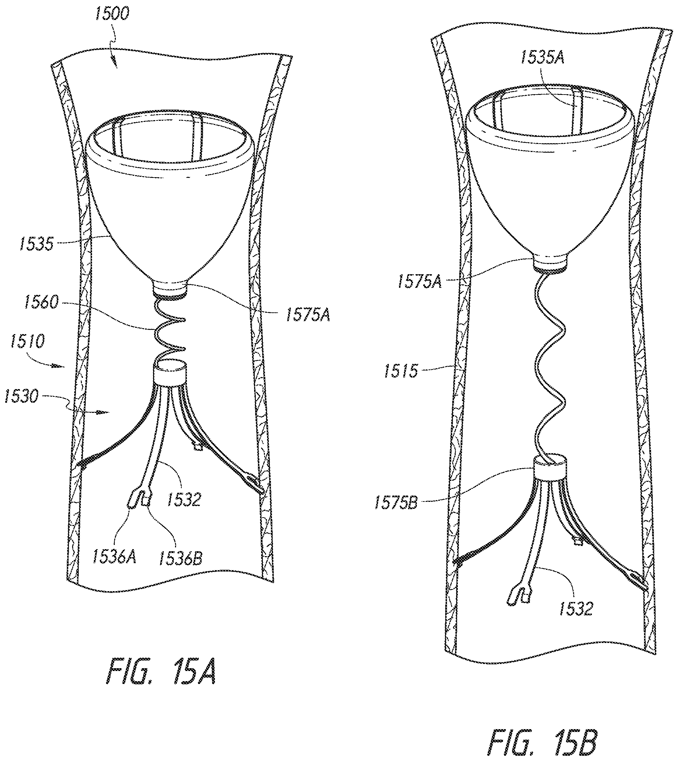

FIG. 15A and 15B show an example of an elastic connecting portion.

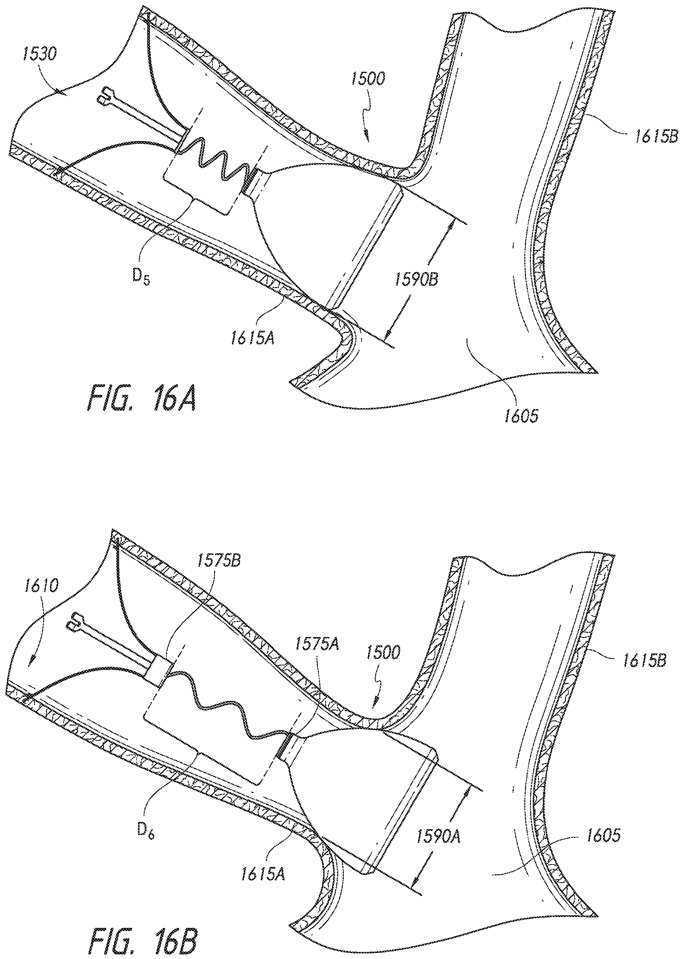

FIG. 16A and 16B show an example of an elastic connecting portion used in a branching lumen.

These and other features will now be described with reference to the drawings summarized above. The drawings and the associated descriptions are provided to illustrate embodiments and not to limit the scope of any claim. Throughout the drawings, reference numbers may be reused to indicate correspondence between referenced elements.

### DETAILED DESCRIPTION OF EMBODIMENTS

Certain anchoring mechanisms for devices used inside airways to lungs can have anchoring struts that can apply an outward force to a lumen (e.g., a lung airway). Such anchoring mechanisms can allow devices to be securely positioned during expansion and contraction of the lumen. Anchoring mechanisms can enter the lumen in a compressed state when devices having valves are being inserted into the lumen. Outward force of the anchoring mechanism can allow the anchoring mechanism to reach its fully expanded state during operation within the lumen. The outward force can maintain a slight outward pressure on the lumen during exhalation.

In some cases, the outward force of the anchors can deform the lumen walls. For example, the outward force can push the lumen walls radially outward from a central axis of the lumen. Expansion of the anchors and/or deformation of the lumen walls can cause an operative portion (e.g., a valve portion) of the device to be pulled towards the anchors (e.g., in a distal direction) as the anchors move outward and away from the axis of the lumen. Such "settling" of the anchors and operative portion of the device can occur gradually or quickly, depending on the geometry, elasticity, and/or other features of the lumen in which the device is implanted.

Pulling of the operative portion during lumen expansion can cause the operative portion to move longitudinally from its initial placement position. The dimensions of the device can be calibrated to account for such movement before placing the device in the lumen. Such calibration can be done by, for example, adjusting the placement of the operative portion (e.g., by placing the operative portion proximal of the intended final location), though a precise adjustment to account for the movement can be difficult. In some cases, movement of the device occurs over a time period beyond which the physician can reasonably observe using a visualization device such as a bronchoscope. In some cases, it can be difficult for the physician to approximate the final resting place of the device. A displacement of a device resulting from the bodily movement can result in unwanted side-branch ventilation due to the sealing portion being moved from its original position, which can compromise the effectiveness of such devices. In some cases, the valve of a device can become overcompressed when moved (e.g., in a distal direction). In some cases, the valve of a device can be moved to a portion of a lumen larger than the valve, thereby reducing or eliminating the efficacy of the valve.

Some mechanisms use stent anchoring designs that can overpower the lumen wall and press against the lumen wall to about the full diameter of the device. Such mechanisms can become affixed to the final resting position in the lumen immediately upon placing the device inside the lumen. However, such mechanisms can present problems over time because those mechanisms may not adjust properly with the expansion and contraction of airways to lungs caused by breathing. The tissue of the lumen may remodel to accommodate the device diameter, possibly causing the device to be loosened and/or coughed out.

Disclosed herein are anchoring mechanisms that can track the movement of a lumen wall (e.g., during breathing and/or coughing) and can allow the sealing portion of a valve to remain at a substantially consistent position in the airway as the anchoring mechanisms transition to a final resting position. In some cases, the anchoring mechanisms apply a vortexly expanding outward force with little or no axial component to the lumen wall. In some cases, the anchoring mechanisms apply a force with an axial vector in the proximail direction to push the device in the proximal direction as the lumen lengthens (e.g., during reexpansion of a collapsed lung).

### Spring-type anchoring mechanism

An anchoring mechanism that can adapt to change in lumen dimensions can be used to place a medical device to a lumen. The medical device can be an endoluminal medical device. The endoluminal medical device used with an anchoring mechanism can include, for example, stents, sensors, valves, etc. The lumen can include, for example, veins, airways, digestive tracts, etc. FIG. 1 shows a schematic transverse cross-sectional view of an example shape of a spring-type anchoring mechanism used inside a lumen. The spring type anchoring mechanism 100 can comprise a hub 120 and a plurality of spring-type anchoring struts 130. The spring-type anchoring struts 130 can comprise an anchoring tip 140.

The hub 120 can be a collet configured to bundle the plurality of spring-type anchoring struts 130. The hub 120 can comprise a centering rod and a forceps knob, not shown. The spring-type anchoring struts 130 can be arranged radially from the hub 120. The spring-type anchoring struts 130 can comprise coiled springs, bent springs, or other expandable geometries and configurations. The anchoring struts 130 used in the spring-type anchoring mechanism 100 can comprise an elastic structure configured to expand or contract according to the movement of the lumen. For example, the plurality of anchoring struts 130 can comprise an expansion spring, such as the spring-type anchoring mechanism shown schematically in FIG. 1. In some embodiments, the anchoring struts 130 are formed as compression springs.

The anchoring tip 140 can be configured to removably attach to the lumen 110. In some embodiments, the anchoring tip 140 can be configured to penetrate a portion of the lumen 110. For example, the anchoring tip 140 can comprise a tapered shape. The anchoring tip 140 can comprise a first piercing mechanism. The first piercing mechanism can comprise a shank. The anchoring tip 140 can comprise an anchor projection 150. The anchor projection 150 can comprise a second piercing mechanism forming an angle with the spring-type anchoring struts 130. For example, as shown schematically in FIG. 1, the anchor projection 150 can form about a 90 degree angle from the radius of the lumen 110. The anchor projection 150 can form an angle less than 90 degrees, about 75 degrees from the radius of the lumen, for example. The anchor projection 150 can comprise a tack. The anchor projection 150 can be configured to allow the anchoring tip to penetrate a portion of the lumen 110. For example, the anchor projection 150 can have a length equal to or less than about 2 times the diameter of the anchoring struts.

In some embodiments, the anchor projection 150 is configured to limit degree of penetration of the anchoring tip 140. For example, the anchor projection 150 can comprise an additional pad portion pointing in a vertical (e.g., circumferential) direction with respect to the hub 120. The anchor projection 150 can be positioned to rest against an inner surface of the lumen 110 to inhibit or prevent penetration of the anchoring tip 140 beyond a predetermined distance.

Dimensional changes of the lumen 110 can include changes in longitudinal dimension and changes in radial dimension. The lumen 1 10 can comprise a largest fully expanded diameter and a smallest fully contracted diameter. The plurality of spring-type anchoring struts 130 can extend radially from the hub 120 to contact the lumen 1 10. Each of the struts 130 can have an extended length (e.g., a length to which the struts 130 extend if unobstructed). In some embodiments, the extended length of the struts is greater than an expanded radius of the lumen 110 in which the struts 130 are deployed. In some embodiments, the extended length of the struts 130 is greater than 1.1 times, greater than 1.25 times, greater than 1.4 times, greater than 2 times, and/or greater than 4 times the expanded radius of the lumen 100. Many variations are possible.

The length of the plurality of spring-type anchoring struts 130 can comprise a coiled length (e.g., a minimum length the struts 130 can assume when the struts 130 are compressed). In some embodiments, the coiled length of the struts 130 is less than the radius of the lumen 110 in the smallest fully contracted diameter of the lumen 110. For example, the coiled length of the struts 130 can be less than about 0.9 times the contracted diameter, less than about 0.75 times the contracted diameter, less than 0.6 times the contracted diameter, and/or less than about 0.5 times the contracted diameter of the lumen 100. Many variations are possible.

The spring type anchoring mechanism 100 can comprise an even number of spring-type anchoring struts 130. For example, as shown in FIG, 1, the spring type anchoring mechanism 100 can comprise six spring-type anchoring struts 130. An even number of spring-type anchoring struts 130 can be used, for example, to counteract expansion and/or contraction forces of opposing spring-type anchoring struts 130. In some embodiments, the anchoring mechanism 100 includes an odd number of anchoring struts 130.

The spring-type anchoring struts 130 can comprise varying lengths. For example, two or more spring-type anchoring struts 130 can have an equal length and a third spring-type anchoring strut 30 can have a length greater than or equal to the other two. In some cases, each of the struts 130 has a different length.

The hub 120 can be disposed on or near the longitudinal axis of the lumen 110 when the anchoring mechanism 100 is deployed. The spring-type anchoring struts 130 can at least partially uncoil during expansion of the lumen 110 and maintain contact with the lumen 110. The spring-type anchoring struts 130 can coil during contraction of the lumen 110 and apply a radial outward force to the lumen 110.

The struts 130 can be configured to maintain the hub 120 in a relatively stable position along a length of the lumen 110 in which the anchoring mechanism 100 before and/or after settling (e.g., expansion of the airway due to outward force of the struts 130) of the anchoring mechanism 100. The longitudinal movement (e.g., movement along a length of the lumen 110) of the hub 120 during and/or before settling can be limited to be within about 1 mm. The movement of the hub 120 can be limited to be, within about 2 mm, within about 0.5 mm, within about 0.3 mm, and/or within about 0.2 mm during and/or before settling.

A user may deploy the anchoring mechanism 100 by using a delivery device, such as an endoscope. The spring type anchoring mechanism 100 can be folded and transported within a catheter used with an endoscope. The spring type anchoring mechanism 100 can be deployed by being pushed out of the catheter. In some embodiments, the anchoring mechanism 100 can be deployed by withdrawing the catheter from the anchoring mechanism 100 at a deployment site. The spring-type anchoring struts 130 can expand radially outward to contact the lumen 110 once placed outside the catheter. The spring-type anchoring struts 130 can exert a radial outward force on the lumen wall 110. The radial outward force can allow each of the anchoring tips 140 to penetrate the lumen 110.

The hub 120 and the anchoring tip 140 can be on the same or substantially same transverse plane upon deployment. In some embodiments, the anchoring mechanism 100 can comprise two or more anchoring struts 130. The spring-type anchoring struts 130 can comprise a combination of elastic and static anchoring struts. For example, the spring-type anchoring struts 130 can comprise two spring-shaped elastic anchoring struts and one stiff, static anchoring strut. The hub 120 can comprise an axial bearing. The hub 120 can be positioned off-center. For example, the hub 120 can be located adjacent to wall of the lumen 1 10. The hub 120 can comprise various different shapes. For example, the hub can have a round, polygonal, oval-shaped, or other-shaped cross-section in a plane perpendicular to a longitudinal axis of the lumen 110. The anchoring tip 140 can comprise a blunt tip, such that no portion of the anchoring tip penetrates the lumen 1 10, The anchoring tip 140 having a blunt tip can comprise a traction surface. For example, the traction surface can comprise a plurality of cleats.

### Spiral-type anchoring mechanism

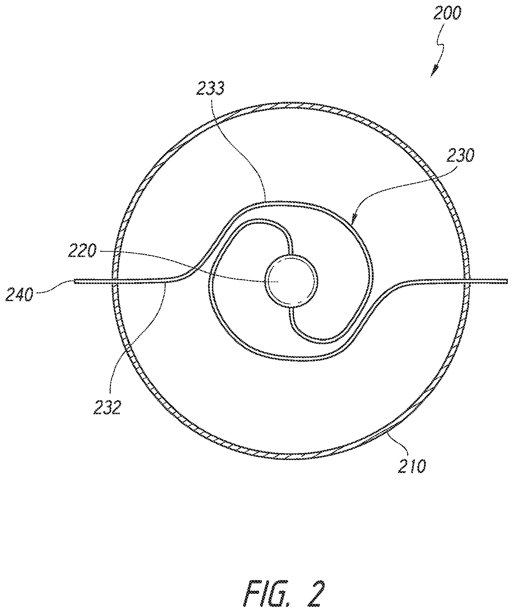

Anchoring struts can comprise a spiral shape, or a torsion spring shape. The spiral shape can be used, for example, to exert torsional force during expansion and contraction of the lumen. FIG. 2 shows a schematic transverse cross-sectional view of an example shape of a spiral type anchoring mechanism 200. The spiral type anchoring mechanism 200 can comprise a hub 220, a plurality of anchoring struts 230, and anchoring tips 240. The plurality of anchoring struts 230 can comprise an elongate (e.g., generally straight) portion 232 and a spiral portion 233. The anchoring tips 240 can be configured to penetrate the lumen 210.

The hub 220 can be placed on or near the longitudinal axis of the lumen 210. The hub 220 of the spiral type anchoring mechanism 200 can remain substantially in the same axial position irrespective of the movement of the lumen. The hub 120 can rotate as the spirals expand. For example, the hub 120 can rotate independently from an operative portion of the medical device during lumen expansion or contraction, in some embodiments, the hub 120 is rotatably locked with and connected to the operative portion. The operative portion can be, for example, a lung airway valve.

During an expansion and contraction of the lumen 110, the spiral type anchoring mechanism 200 can inhibit, reduce, or prevent displacement of a medical device while allowing the hub 220 to remain substantially at its original position. For example, during expansion of the lumen, the spiral portion 233 of the anchoring struts 230 can straighten. The hub 220 can remain suspended from the lumen wall and remain substantially at its original position. In some embodiments, the anchoring mechanism 200 is configured to maintain the hub 200 (e.g., and, consequently, the operative portion) in a relatively constant position along a length of the lumen 210 in which the mechanism 200 is deployed.

The plurality of anchoring struts 230 can be made of shape memory material, such as Nitinol. In some embodiments, the struts 230 are constructed from another, resilient and/or flexible material. The plurality of anchoring struts 230 can comprise a coiled length. The coiled length can comprise the shortest length between the hub 220 and the end of the anchoring tip 240 when the struts 230 are radially compressed. In some embodiments, the coiled length of the struts 230 is less than about 0.9 times, less than about 0.75 times, less than about 0.6 times, less than about 0.4 times, and/or less than about 0.25 times a radius of the lumen 210. The plurality of anchoring struts 230 can comprise an uncoiled length larger than a radius of lumen 210. For example, the straight length of the anchoring struts 230 can be more than 2 times larger, more than 1.5 times larger, and/or more than 1.1 times larger than a radius of the lumen 210.

The spiral shapes anchoring mechanism 200 can be transported across a catheter by being folded into the catheter prior to deployment. The anchoring struts 230 can be coiled further to be transported through the catheter. The spiral shape anchoring mechanism 200 can uncoil to its pre-formed spiral shape when pushed out of the catheter, in some embodiments, the anchoring mechanism 200 uncoils upon withdrawal of the catheter from the struts 230 at a deployment site in the lumen 210. The anchoring tips 240 can contact the lumen 210 once deployed, and exert an outward force from the hub 220 to the lumen 210. The anchoring tips 240 can penetrate the flesh of the lumen 210 by exerting outward force. In some embodiments, the anchoring tips 240 include pads or other mechanisms configured to limit the depth to which the tips 240 penetrate the lumen wall.

The anchoring struts 230 can be straightened to be transported through the catheter prior to deployment. The hub 220 can be a dual hub configured to allow rotation of the hub 220 without rotating the attached portion of the medical device (e.g. the operative portion). The spiral shaped anchoring mechanism 200 can comprise three or more anchoring struts 230. The anchoring struts 230 can comprise a combination of different spring shapes. For example, the anchoring struts 230 can comprise a plurality of spring-type anchoring struts 130 (e.g., shown in FIG. 1) helically coiled and forming a spiral around the hub 220.

### Vortex-type anchoring mechanism

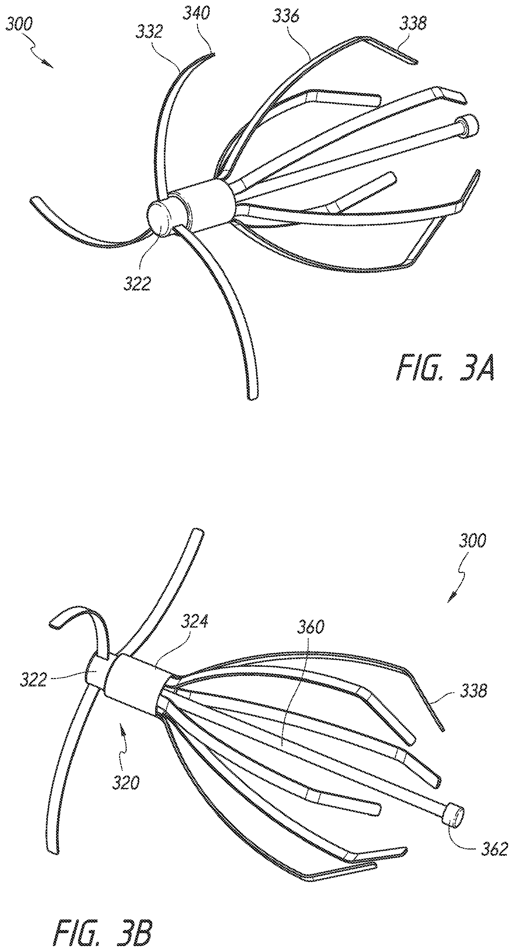

An anchoring mechanism can comprise a plurality of flexible struts that are bent in a same circumferential direction to form a vortex shape when viewed in a transverse cross-section. The flexible vortex struts can bend or straighten, bending further during contraction of the lumen and straightening as the lumen expands. FIGS. 3A and 3B show perspective views of an example shape of a vortex-type anchoring mechanism. The vortex-type anchoring mechanism 300 can comprise a hub 320, a plurality of flexible anchoring struts 332, a plurality of proximal struts 336, a center rod 360, and/or a forceps knob 362. The hub 320 can comprise a dual-hub configuration having a distal hub 322 and a proximal hub 324. The flexible anchoring struts 332 can comprise an anchoring tip 340. The proximal struts 336 can comprise a bent contact surface 338. The proximal struts 336 can comprise frame struts of an operative portion 1135 (e.g., shown in FIGS. 12A to 12C).

The flexible anchoring struts 332 can extend radially outward from the distal hub 322. The proximal hub 324 can be connected to and/or form a monolithic part with the distal hub 322. The proximal struts 336 can extend radially and proximally from the proximal hub 324. The proximal struts 336 can form a basket. The proximal hub 324 can be a collet. The center rod 362 can be connected to the proximal hub 324 and extend proximally from the center hub. The center rod 362 can connect the hub 320 and the forceps knob 362. The forceps knob 362 can be shaped and sized to be grasped by a forceps. The plurality of flexible anchoring struts 332 can be configured to radially expand and exert an outward force on the lumen. The bent surface 338 of the plurality of proximal struts 336 can comprise portions of the proximal struts that bend at an angle from the rest of each proximal strut member 336 towards the longitudinal axis, or the center rod 360. The bent contact surface 338 can be used, for example, to inhibit or prevent trauma to the lumen wall by the proximal struts 336.

The distal hub 322 and the proximal hub 324 can comprise collinear cylindrical structures. The distal hub 322 and the proximal hub 324 can comprise cylindrical structures having different diameters. For example, the diameter of the distal hub 322 can be smaller than the diameter of the proximal hub 324. The smaller diameter of the distal hub 322 can be shaped and sized, for example, to allow the plurality of distal struts to coil and fit inside a delivery catheter. Each of the plurality of proximal struts 336 can have a straightened length. The straightened length can comprise the length of the proximal strut member at a fully straightened state with no bends. The straightened length can be less than 1.5 times the length of the center rod 362. The straightened length can be equal to or less than the length of the center rod 362. in some cases, the straightened length can be greater than 1.5 times the length of the center rod 362.

The stiffness of the flexible anchoring struts 332 can be tuned to counteract the forces (e.g., tension and compression) occurring during expansion of the struts 332. For example, stiffness of some portions in the struts 332 may be greater than the stiffness in other portions. Areas near the hub 320 and near the anchor tip 340 can be relatively stiff, while near the middle of the struts can be made more flexible to allow bending. The stiffness can be tuned by changing dimensions and/or shape of portions of the anchoring struts 332. For example, the plurality of anchoring struts 332 can comprise bent portions, thin portions, thick portions, and/or spiral portions.

The vortex shape anchoring mechanism 300 can be folded and transported across a delivery catheter. The flexible anchoring struts 332 can be coiled in the direction of the bend to fold and fit inside a catheter. For example, the flexible anchoring struts 332 shown in FIGS. 3A to 3B can be coiled by rotating the hub 320 relative to the anchoring tip 340 in a counterclockwise direction. The proximal struts 336 can be straightened to fold and fit inside a catheter. The proximal struts 336 can be straightened along the direction of the length of the center rod 362.

The flexible anchoring struts 332 in a fully coiled state can have a cross-sectional diameter equal to or less than 1 .5 times larger than the diameter of the proximal hub. The flexible anchoring struts 332 in a fully coiled state can have a cross-sectional diameter equal to or less than the diameter of the proximal hub. The plurality of proximal struts 336 in a fully straightened state can have a cross-sectional diameter equal to or less than 1.5 times larger than the diameter of the proximal hub. The plurality of proximal struts 336 in a fully straightened state can have a cross-sectional diameter equal to or less than the diameter of the proximal hub.

The vortex strut anchoring mechanism 300 can counteract movement of the lumen. For example, the vortex strut anchoring mechanism 300 can adjust to expand during lumen expansion and contract during lumen contraction. The anchoring tips 340 can remain in a substantially same transverse plane (e.g., perpendicular to the centerline of the lumen) during lumen expansion and contraction. For example, the flexible anchoring struts 330 can be bent further to have a smaller diameter, such that the anchoring tip 340 and the hub 320 can remain in a substantially constant transverse plane. The arc of the curvature of the anchoring struts 332 can straighten more as the lumen expands, while contact points and the hub remain in substantially the same plane. The stiffness of the anchoring struts can be tuned to counteract distal and proximal forces applied when the anchoring struts 330 expand. In some embodiments, the struts 332 are configured to remain in substantially the same transverse plane during and/or after settling of the device 300.

The plurality of proximal struts 336 can comprise two or more bent surfaces 338. For example, a proximal strut 336 can have two connected bent surfaces that form two included angles from the proximal strut 336. Two or more bent surfaces 338 can be used, for example, to allow different atraumatic contact surfaces to contact the lumen during expansion and contraction. For example, bent surfaces 338 can comprise a first surface configured to contact the lumen at an expanded state, and a second surface configured to contact the lumen at the contracted state.

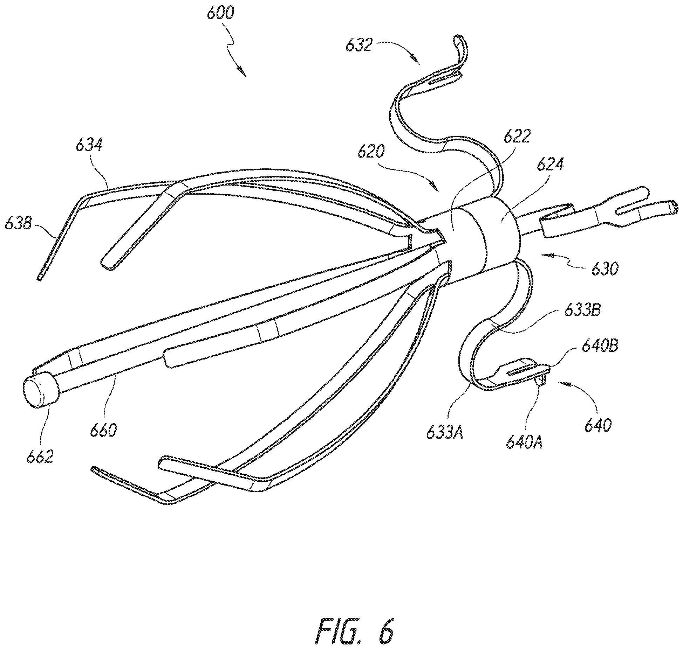

The plurality of flexible anchoring struts 332 can comprise anchoring tips 340. For example, the plurality of flexible anchoring struts 332 can comprise multiple anchoring structures pointing in different directions. For example, the anchoring tip 340 can comprise a bi-directional anchoring tip 640 (shown in FIG. 6). In some embodiments, the anchor tips 340 comprise atraumatic and/or high friction tips configured to engage with the wail of a lumen. The plurality of flexible anchoring struts 332 can comprise a spiral shape, such as the spiral-type anchoring struts 230 shown and described above in reference to FIG. 2. The proximal struts 336 can be used to counteract movements of the attached medical device in the proximal direction.

The position of the proximal struts 336 and the flexible anchoring struts 330 can be reversed. For example, the proximal struts 336 can be positioned distally relative to the flexible anchoring struts 330. The proximal struts 336 can extend in the opposite direction, such that the bent contact surface 338 can be positioned distally relative to the hub 320. The vortex strut anchoring mechanism 300 can comprise different number of flexible anchoring struts 330 as the number of proximal struts 336. The vortex strut anchoring mechanism 300 can comprise the same number of flexible anchoring struts 330 as the number of proximal struts 336. For example, where the vortex strut anchoring mechanism 300 has three flexible anchoring struts 330, the mechanism 300 can comprise three proximal struts 336. In some embodiments, each of proximal struts 336 and each of flexible anchoring struts 330 can be connected and can comprise portions of singular strut members. For example, a plurality of singular strut structures can be bundled together between the two ends of each strut structure by a collet.

### Wave-type anchoring mechanism

According to the invention anchoring mechanism 400 includes struts having a wave-like shape when viewed from a longitudinal cross-sectional view of the lumen. FIGS. 4A and 4B show a schematic drawing of an example shape of a wave-type anchoring mechanism 400. The wave-type anchoring mechanism 400 includes a hub 420, a center rod 460, and a plurality of undulating anchoring struts 430. The undulating anchoring struts 430 can comprise one or more thick portions 433A, one or more thin portions 433B, and anchoring tips 440. The undulating anchoring struts 430 comprise a substantially straight shape when viewed in a transverse direction. The wave-type anchoring mechanism 400 can limit displacement of the hub 420 as the lumen 410 shifts between a contracted state (FIG. 4A) and an expanded state (FIG. 4B). In some cases, the anchoring mechanism 400 limits displacement of the hub 420 (e.g., and, consequently, an operative portion connected to the hub 400) when the mechanism 400 settles in the membrane 450.

The hub 420 can connect the plurality of undulating anchoring struts 430 to the center rod 460. For example, the wave-type anchoring mechanism 400 can comprise two undulating anchoring struts 430 connected to the center rod 460 via the hub 420. In some embodiments, the anchoring mechanism 400 includes three or more anchoring struts 430 emanating from the hub 420. The undulating anchoring struts 430 can comprise one or more inflection points and undulation points. For example, as shown in FIG. 4A and 4B, a wave-type anchoring mechanism 400 can have at least one undulation point on each strut 433.

The anchoring tip 440 can be located proximally to the location of the hub 420 at a distance D1 when the anchoring struts 430 are in a first, less-expanded configuration. For example, the distance between the anchoring tip 440 and the hub 420 in a fully contracted state lumen (FIG. 4A) can comprise a first distance D1. The distance between the anchoring tip 440 and the hub 420 in a fully expanded state lumen (FIG. 4B) can comprise a second distance D2. The displacement of the hub 420 can be limited such that the difference between the first distance D1 and the second distance D2 can be less than 0,5 mm. The difference between the first distance D1 and the second distance D2 can be less than .1 mm. The difference between the first distance D1 and the second distance D2 can be less than .05 mm. In some cases, the difference between the first distance D1 and the second distance D2 can be less than. 02 mm.

The anchor struts 430 can have non-uniform thickness between the hub 420 and the anchor tips 440. For example, the anchor struts 430 can include a thick portion 433A which can be more rigid than a thinner portion (e.g. having a greater degree of freedom). For example, the thick portion 433A can retain its bent shape while experiencing deflection during expansion of the lumen 410 and/or during settling of the anchoring mechanism 400. The anchoring mechanism 400 can include a thin portion 433B may deflect to a greater degree than the thick portion 433A during expansion of the lumen 410. The thick portion 433A and the thin portion 433B can comprise portions of undulating anchoring strut 430 having different structures and dimensions. For example, the thick portion 433A can comprise a portion of the strut member 430 having a greater thickness along a direction transverse to the longitudinal direction of the lumen 410 while the thin portion 433B comprises a strut portion having a greater thickness along a longitudinal direction of the lumen 410. Such a configuration can be used to allow the thick portion 433A. to withstand stress from repeated expansion and contraction motion of the lumen 410.

As shown in FIGS. 4A and 4B, the wave-type anchoring mechanism 400 can comprise an anchoring tip 440. The anchoring tips 440 can be curved and pointing at a distal direction. The wave-type anchoring mechanism 400 can be placed proximally or distally of an operative portion of a medical device, such as a valve.

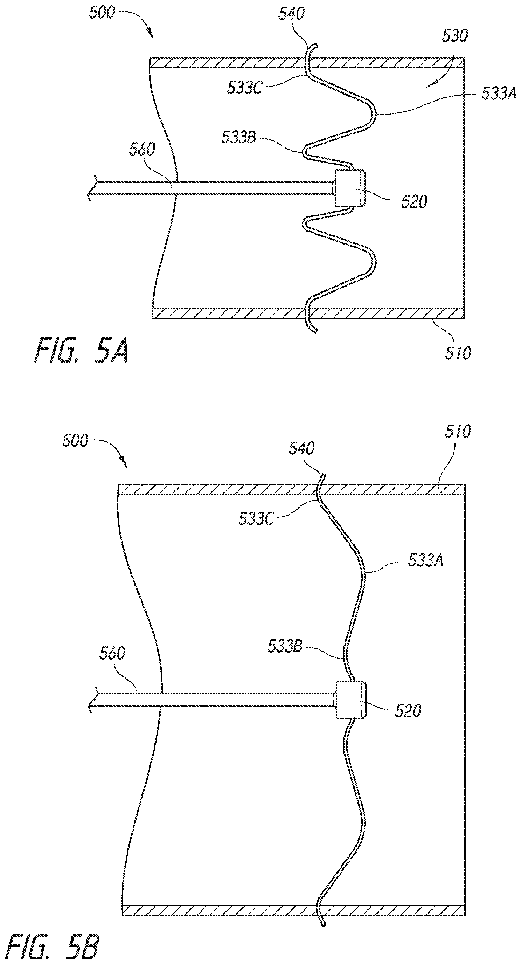

The undulating anchoring struts 430 can comprise struts having different lengths, shapes, points of inflection/undulation, stiffness, etc. For example, FIGS. 5A and 5B show an example shape of a wave-type anchoring mechanism having a bow-shaped bend near the hub 520. The wave-type anchoring mechanism 500 having a sharper bend near the hub 520 can comprise a center rod 560, a hub 520, and a plurality of anchoring struts 530. The plurality of anchoring struts 530 can comprise a thick portion 533A, a thin portion 533B, a bow-shaped bend 533C, and anchoring tips 540. The bow-shaped bend 533B can comprise a different structure from the remainder of the anchoring struts 530. For example, the bow-shaped bend 533B can comprise a bent shape in a transverse direction.

The anchoring tip 440 can be located on the same transverse plane as the hub 420. The anchoring tip 440 can point in a proximal or distal direction. In some embodiments the anchoring tip 440 includes a pad portion configured to limit a depth to which the anchoring tip 440 penetrates the lumens 410. The wave -type anchoring struts 430 can extend radially from the hub 420. For example, FIG. 6 shows a perspective view of an example shape of a wave-type anchoring mechanism 600 comprising undulating struts 632. The anchoring mechanism 600 can share many or most of the characteristics and features of the anchoring mechanism 300 described above, wherein the distal anchors 632 comprise undulating struts rather than the vertical struts 332 described above, and wherein like parts include like numbers (e.g., proximal struts 336 v. proximal struts 636). The hub 620 can comprise a proximal hub 622 and a distal hub 624. The plurality of vortex undulating struts 632 can comprise a thick portion 633A, a thin portion 633B, and a bi-directional anchoring tip 640. The bi-directional anchoring tip 640 can comprise a first anchoring tip 640A and a second anchoring tip 640B. The second anchoring tip 640B can be configured to limit the extent to which the first anchoring tip 640A.

As shown in FIG. 6, the wave-type anchoring mechanism comprising undulating struts 600 can comprise three or more undulating struts 632. The bidirectional tip 640 can comprise the bi-directional tip 1335A, 1335B shown and described in reference to FIGS. 13A to 13C of this application.

### Diamond-shaped anchoring mechanism

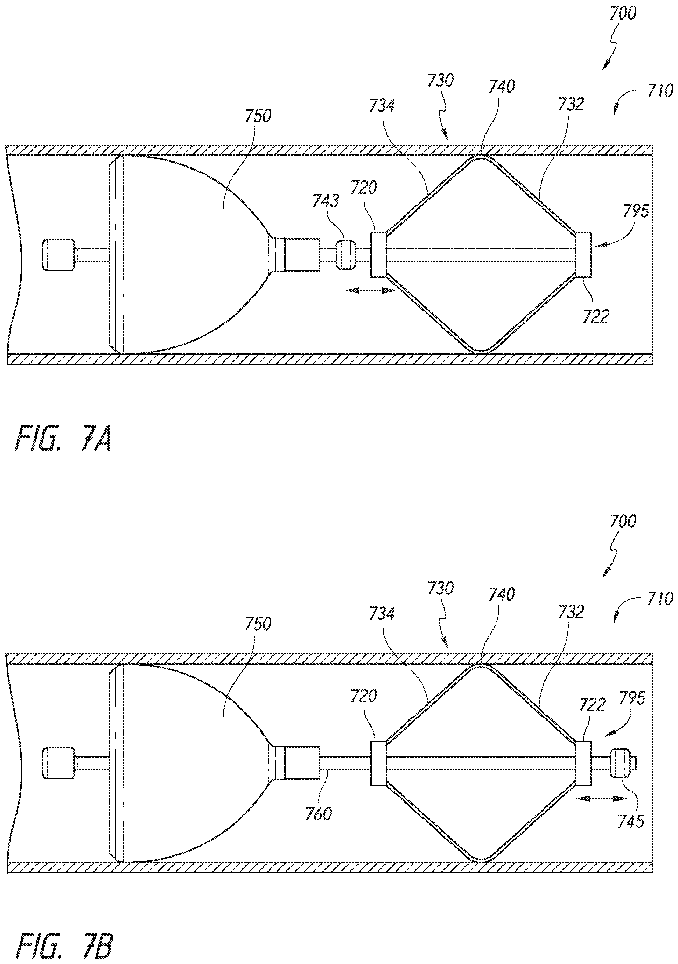

FIG. 7A and 7B schematically show examples of a diamond shaped anchoring mechanism. The diamond shaped anchoring mechanism 700 can comprise a forceps knob 762, a center rod 760 extending distally from the forceps knob 762, a first hub 720 connected to the center rod 760 distal from the forceps knob 762, a second hub 722 connected to the center rod 760 distal of the first hub 720, and/or a plurality of diamond-shaped anchoring struts 730. The plurality of diamond-shaped anchoring struts 730 can comprise a proximal portion 734, a distal portion 732, a distal point 795, and anchoring tips 740. In some embodiments, an operative portion 750 is connected to the center rod 760 between the first hub 720 and the forceps knob 762.

The center rod 760 can connect the forceps knob 762 and the distal point 795. For example, the distal point of the struts 730 can be connected to the second hub 722. The proximal portion 734 and the distal portion 732 can comprise two sides of a diamond. The proximal portion 734 and the distal portion 732 can comprise a singular strut member wherein the proximal portion 734 is connected to the first hub 720.

The proximal portion 734 and the distal portion 732 can form an included angle. For example, in a first state (e.g. before settling of the device 700 and/or before expansion of the lumen 710), the proximal portion 734 and the distal portion 732 can form an angle less than about 150 degrees. The proximal portion 734 and the distal portion 732 can form an angle less than about 80 degrees in the original state. The proximal portion 734 and the distal portion 732 can form an angle less than about 60 degrees in the original state. The proximal portion 734 and the distal portion 732 can form an angle less than about 45 degrees in the original state.

The anchoring tip 740 can comprise an anchoring structure. For example, the anchoring tip can comprise a barb, high friction pad, or other structure configured to penetrate and/ or frictionally engage with the lumen 710. The anchoring tip 740 can comprise a bi-directional anchoring tip. For example, the anchoring tip 740 can comprise bi-directional tip shown and described in reference to FIGS. 13A to 13C of this application, ḯη some embodiments, the anchoring tip 740 can comprise cleats, studs, tracks, etc.

As shown in FIGS. 7A-7B, when viewed in a longitudinal cross section, the diamond-shaped anchoring struts 730 can comprise a diamond shape, or two mirroring triangles. A proximal end of the triangles can be connected to the first hub 720.I n some embodiments the distal end of the triangles is connected to the second hub 722. The struts 730 can be configured to expand and/or contract in a radial direction in reaction to expansion and contraction of the lumen 710 (e.g., due to breathing, settling of the device 700, or other circumstances).

The struts 730 can be configured to expand/contract in a radial direction with limited or no movement of the center rod 760 along a length of the lumen 710. For example, the first hub 720 can be a sliding hub (FIG. 7A). The hub 720 can comprise an annular shape with a hollow center shaped and sized to slide along the center rod 760. The hub 720 (e.g., and the proximal end of the struts 730) can be configured to slide in a distal direction when the struts 730 expand. The mechanism 700 can include a proximal stop 743 configured to limit the proximal movement of the first hub 720 along the center rod 760.

In some embodiments the second hub 722 is configured to slide along the center rod 760 (FIG. 7B). In some cases, the second hub 722 is slidable and the first hub 720 is fixed on the center rod 760. The second hub 722 (e.g., and the distal end of the struts 730) can be configured to slide in a proximal direction then the struts 730 expand. In some embodiments, the mechanism 700 includes a distal stop 745 configured to limit distal movement of the second hub 722 when the second hub 722 is a sliding hub (FIG. 7B).

In some cases, sliding of the first hub 720 or second hub 722 can permit radial expansion and contraction of the struts 730 (e.g., in reaction to inhalation/exhalation and/or settling of the mechanism 700) with little or no movement of the center rod 760 along a length of the lumen 710. Limiting or preventing movement of the center rod 760 along the length of the lumen 710 can facilitate constant or semi-constant positioning of the operative portion 750.

The proximal portion 734 and the distal portion 732 can have approximately equal lengths. In some embodiments, the proximal portion 734 and the distal portion 732 comprise different lengths. The diamond-shaped anchoring mechanism 700 can comprise a telescopically sliding rod. For example, a sliding rod can slidably connect the center rod 760 to the distal point 795. The center rod 760 can comprise a hollow center shaped and sized to accept the sliding rod.

### Bowtie-shaped anchoring mechanism

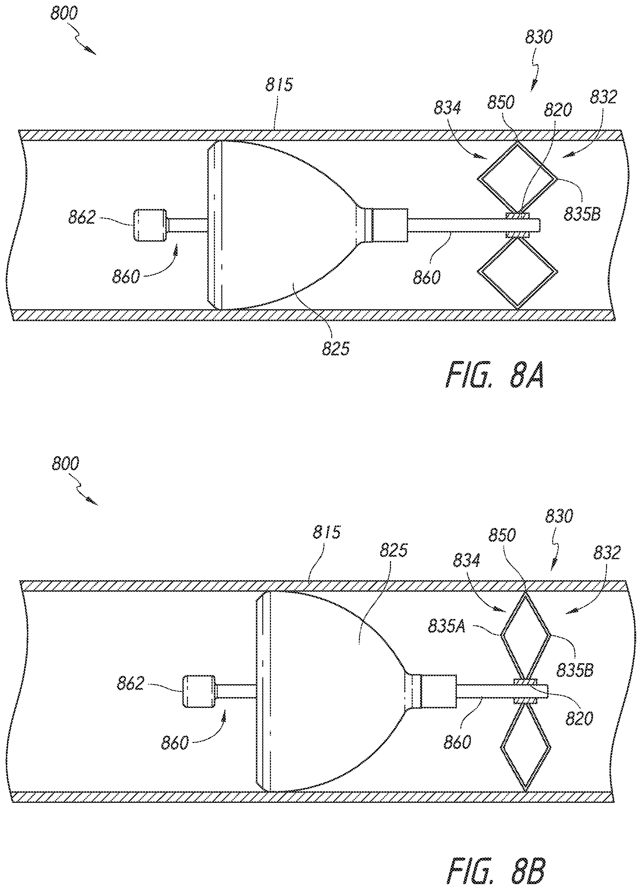

FIGS. 8A and 8B show an example shape of an anchoring mechanism having bowtie-shaped anchoring struts 834 used with an operative portion 825. The bowtie-shaped anchoring mechanism 800 can comprise a forceps knob 862 and a center rod 860 connected to and extending distally from the forceps knob 862. An operative portion 82.5 (e.g., valve) can be connected to the center rod 860 distal from the forceps knob 862. The mechanism 800 can include a hub 820 connected to the center rod 860 distal of the operative portion 825.

As illustrated, the mechanism 800 can include a plurality of bowtie-shaped anchoring struts 830. The struts 830 can be connected to and extend radially from the hub 820. The bowtie-shaped anchoring struts 830 can comprise a plurality of quadrilateral- shaped members. The struts 830 can each comprise a proximal portion 834, a distal portion 832, and anchoring tips 850. The proximal portion 834 can comprise a proximal point 835A. The distal portion 832 can comprise a distal point 835B. The proximal and distal points 835A, 835B can comprise bendable or articulated joints. The bowtie-shaped anchoring struts 800 can be used with an operative portion 825, such as a valve used in a lung airway valve.

The shape of the bowtie-shaped anchoring struts 830 can change during movement of the lumen 815 (e.g., during inhalation/exhalation and/or during settling of the mechanism 800). For example, the distance between axis of the lumen 815 and the anchoring tips 850 can grow larger during expansion of the lumen wall. The proximal point 835A and the distal point 835B can move away from each other during contraction of the lumen 810. The proximal point 835A and the distal point 835B can move closer during expansion of the lumen 810.

In some embodiments, the points 835A, 835B of each strut 830 lie on a plane substantially parallel a longitudinal axis of the lumen 815. In some cases, the points 835A, 835B of each strut 830 lie on a plane substantially perpendicular to the longitudinal axis of the lumen 815. In some embodiments, the points 835 A, 835B of each strut 830 lie on planes neither perpendicular nor parallel to the longitudinal axis of the lumen 815. Many variations are possible.

The struts 830 can be configured to expand and/or contract with little or no movement of the hub 820 (e.g., and, consequently, the operative portion 825) along a length of the lumen 815. For example, radial movement of the anchor portions 850 of the struts 830 can occur on a substantially constant plane perpendicular to a length of the lumen 815.

### Opposing anchoring struts mechanism

In some cases, an anchoring mechanism can include one or more anchors configured to engage with a lumen wall to inhibit or prevent movement of an operative portion of a device in a distal direction. For example, two or more anchoring struts can be used to pinch a portion of the lumen.

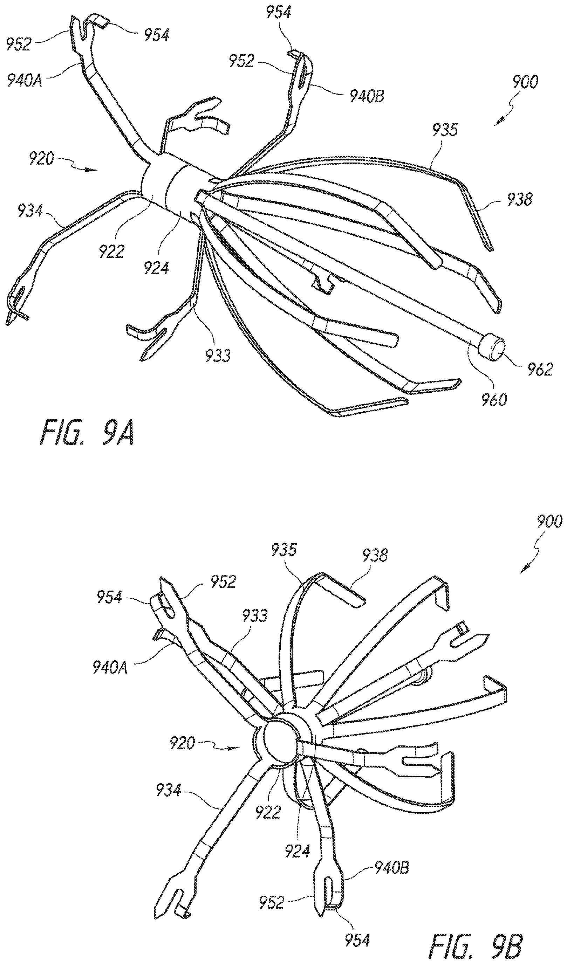

FIGS. 9A and 9B show perspective views of an example shape of a pinch-type anchoring mechanism 900. The mechanism 900 can include a hub 920 and a plurality of distal struts 934 extending distally and/or radially outward from the hub 920. The mechanism 900 can include a plurality opposing struts 933 extending radially outward from the hub 920 proximal of the distal struts 934. The plurality of distal struts 934 can comprise distal anchoring tips 940A. The opposing struts can comprise opposing anchoring tips 940B. The distal and opposing anchoring tips 940A, 940B can comprise bi-directional anchoring tips (shown in FIGS. 13A to 13C).

In some embodiments, the mechanism 900 includes a plurality of proximal struts 935 extending proximally from the hub 920. The plurality of proximal struts 935 can comprise frame struts of an operative portion 1235 (Shown in FIGS. 12A to 12C).

The plurality of distal struts 934 can comprise a strut member comprising one or more bends. For example, as shown in FIG. 9A and 9B, the plurality of distal struts 934 can comprise two bends, each bends curving each distal strut member 934 in a proximal direction. The opposing struts 933 can comprise strut members mirroring the distal struts 934 with respect to a plane perpendicular to a longitudinal axis of the center post 960, For example, the opposing struts 933 can comprise strut members extending radially outward from the hub 920 and initially extending in the proximal direction and bending in the distal direction as shown in FIGS. 9A and 9B. The opposing struts 933 can comprise opposing anchoring tips 940B. The anchoring tips 940A, 940B can comprise a second tip 954. The second tips 954 of the anchoring tips 940A, 940B can be configured to limit a depth to which the anchoring tips 940A, 940B (e.g., or piercing portions 952 thereof) penetrate a lumen wall. For example, the second tip 954 of the proximal anchoring tip 940A can point in the proximal direction, while the second tip 954 of the opposing anchoring tip 940B can point in the distal direction. In some embodiments, the second tips 954 of the anchor tips 940A, 940B point in the same direction.

The hub 920 can comprise a dual-hub configuration, as described above in reference to FIGS. 3A and 3B. The hub can comprise a distal hub 922 and a proximal hub 924. As shown in FIGS. 9A and 9B, the distal struts 934 can extend radially from the distal hub 922. The opposing struts 933 can extend radially from the proximal hub 924.

The opposing struts 933 can be configured to counteract forces created from movement of the pinch-type anchoring mechanism 900 in a distal direction (e.g., due to settling of the mechanism 900). The distal struts 934 and the opposing struts 933 can be configured for use inside a lumen while minimizing pinching of the lumen. Such configuration can be used, for example, to minimize trauma to the lumen.

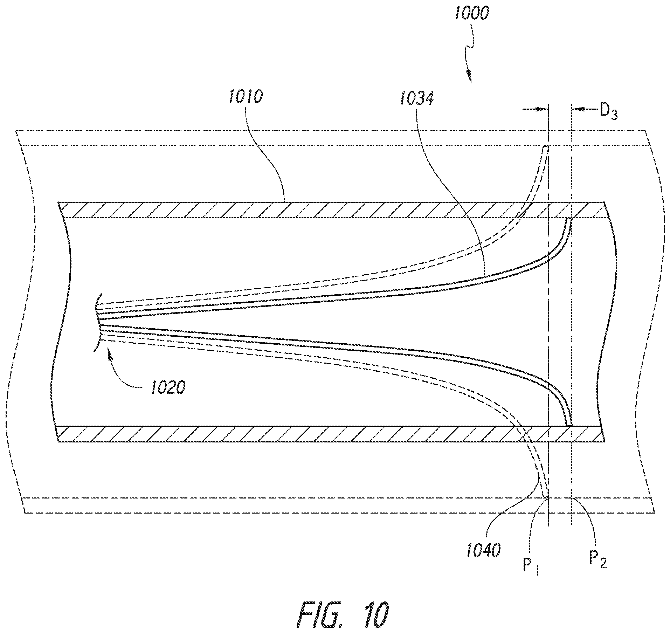

The pinching of the distal struts 934 and the opposing struts 933 can cause the wall of the lumen to become pleated (see, e.g., FIGS. 12A-12C). The pleating of the lumen wall can be used to allow point loads to be applied to wall while permitting use of proximal struts 933 having reduced radial stiffness compared to the distal struts 934. For example, the pleating of the lumen wall can permit the proximal struts 933 to contact and/or penetrate the lumen wall at a smaller angle with respect to the longitudinal axis of the mechanism 900 than the angle of contact when no pleating is present. The pleating of the lumen wall and the minimal opposing forces can be used to inhibit or reduce the likelihood of pressure necrosis which can be caused by the directly opposing forces by the opposing struts. The opposing struts 933 can be shorter than the distal struts 934. The distal struts 934 can comprise elongated anchoring mechanism shown and described in reference to FIG. 10.

The pinch-type anchoring mechanism 900 can be configured to fit inside a delivery catheter. For example, as illustrated in Figures 9A and 9B, the distal struts 934 can be straightened in the distal direction and the opposing struts 933 can be straightened in the proximal direction to fit inside a catheter. A user may place the pinch- type anchoring mechanism 900 at a desired location inside lumen and push the pinch-type anchoring mechanism 900 distally. in some cases, the mechanism 900 is maneuvered to an installation site using a catheter and the catheter is withdrawn from the mechanism 900 to deploy the mechanism at the installation site. Once the distal struts 934 have been released, portions of the distal anchoring tips 940A can connect the pinch-type anchoring mechanism 900 to the lumen and penetrate the lumen. The user may pull the catheter to further release the pinch-type anchoring mechanism 900 such that the opposing struts 933 can be released. The opposing struts 933 can shift shape from a straightened state inside the catheter and return to its original fully curved shape. The opposing struts 933 can form a relatively straight shape within the catheter and can shift shape to curve in the distal direction upon deployment from the catheter. The opposing anchoring tips 940B of the opposing struts 933 can be lodged into the lumen. The deflection force applied to the lumen as the distal struts 934 and the opposing struts 933 return to its respective original shape can pinch the lumen, forming a pleat. In some cases, changes in shape of the pinch-type anchoring mechanism 900 can be triggered or actuated by changes in temperature, by passing an electronic current to the strut members, physical manipulation, etc,

The distal struts 934 and the opposing struts 933 can comprise struts having different lengths, shapes, points of inflection/undulation, stiffness, etc. For example, the distal struts 934 and the opposing struts 933 can comprise strut members having a curved shape, without bends.

### Elongated distal anchors

Anchoring mechanisms having elongated strut members can be used to reduce movement of the medical device caused by bodily movements and/or settling of the anchoring mechanism. The degree of movement of a medical device can be less with anchoring mechanisms using longer strut members, compared to mechanisms using shorter strut members. Longer strut members can expand in a large arc, thereby reducing the axial movement of the endoluminal device as the strut members expand to an equilibrium position (e.g., settle). Fig. 10 schematically shows an example shape of an elongated struts anchoring mechanism. The elongated struts anchoring mechanism 1000 can comprise a plurality of elongated anchoring struts 1034 and a hub 1020.

The elongated anchoring struts 1034 can be configured to reduce or limit the amount of longitudinal movement of a device connected to the hub 1020. The plurality of elongated anchoring struts 1034 can comprise anchoring tips 1040. The anchoring tips 1040 can be in a first location P1 in a fully contracted lumen. The anchoring tips 1040 can be in a second location P2 in a fully expanded state. The anchoring tips 1040 can travel at a longitudinal travel distance D3 between P1 and P2. The longitudinal travel distance D3 can be reduced by having elongated anchoring struts 1040. For example, the longitudinal travel distance D3 can be less than 0.1 mm. The longitudinal travel distance D3 can be less than .05mm. The longitudinal travel distance D3 can be less than .02mm. In some cases, the longitudinal travel distance D3 can be less than .01mm. Many variations are possible.

The elongated anchoring struts 1034 can comprise curved strut members, as shown schematically in FIG. 10. The elongated anchoring struts 1034 can comprise a straight length, the straight length corresponding to the length of strut members when the strut members are straightened. The straight length can be longer than the diameter of the expanded lumen. For example, the elongated anchoring struts 1034 can have a straight length of at least about 1.5 times the diameter of the expanded lumen. The elongated anchoring struts 1034 can have a straight length of at least about two times the diameter of the expanded lumen. The elongated anchoring struts 1034 can have a straight length of at least about three times the diameter of the expanded lumen. The elongated anchoring struts 1034 can have a straight length of at least about four times the diameter of the expanded lumen. In some embodiments, the struts 1034 have a straight length between about one and four times the diameter of the expanded lumen. Many variations are possible.

A user may select shape and dimension of the elongated struts anchoring mechanism 1000 based on the lumen 1010. The straight length and the curvature of the elongated anchoring struts 1034 can be adjusted to correspond to lumen size changes. For example, for a lumen that shows little change in size between its fully expanded state and fully contracted state, strut members having a greater degree of curvature, and/or having a shorter straightened length, can be used. A user may also calibrate the distance traveled by the hub 1020 by adjusting the straight length and/or the curvature. The user can limit the amount of longitudinal distance traveled by the hub 1020 to be less than .02 mm by selecting struts 1040 having a certain length, for example.

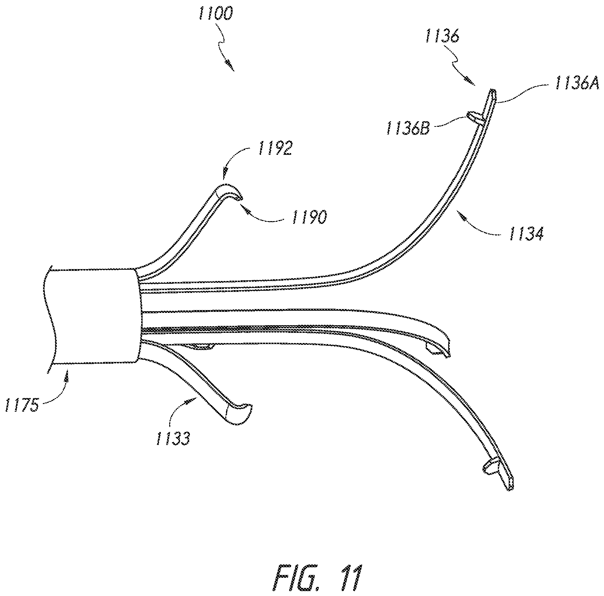

The elongated anchoring struts 1034 can comprise strut members of different lengths and configurations. FIG. 11 shows an example shape of a pinch-type anchoring mechanism 1100 having a plurality of elongated distal anchor struts 1134. The pinch-type anchoring mechanism 1100 can comprise a hub 1175. The mechanism 1100 can include plurality of elongated distal anchoring struts 1134 connected to and extending from the hub 1175 in a distal direction. The mechanism 1100 can include a plurality of opposing struts 1133 connected to the hub 1175 and positioned proximal to the distal struts 1134. The elongated distal anchoring struts 1134 can comprise a distal anchoring tip 1136. The distal anchoring tip 1136 can comprise a first anchoring tip 1136A and a second anchoring tip 1136B. The plurality of opposing anchors can comprise an opposing anchor tip 1190.

As illustrated in FIG. 11, the opposing struts 1133 can extend radially outward from the hub 1175 and extend in a distal direction. The opposing anchor tip 1190 can extend at an angle from the opposing struts 1133. The opposing anchor tip 1190 can form an angle between about 90° and 170° from the opposing struts 1 133. The first anchoring tip 1 136A of the elongated distal anchoring struts 1134 can extend in the same direction as the elongated distal anchoring struts 1134. The second anchoring tip 1136B can point in the proximal direction and form an angle with the first anchor 1136A. The angle can be about 30 degrees to about 170 degrees. The angle can be about 45 degrees to about 150 degrees. The angle can be about 60 degrees to about 120 degrees. The angle can be about 80 degrees to about 100 degrees. The angle can be about 90 degrees. In some embodiments, the second anchoring tip 1136B is configured to interface with a wall of the lumen and limit a depth to which the first anchor tip 1 136A pierces the lumen wall.

In some embodiments, the distal anchoring tip 1136 can comprise a bidirectional anchoring tip (e.g., as shown in FIGS. 13A to 13C). The distal anchoring tip 1136 and the opposing anchor tip 1190 can be configured to pinch a lumen wall. For example, the elongated distal anchoring struts 1136 and the opposing struts 1133 can be configured to oppose each other to pinch a lumen wall. In some embodiments, the opposing struts 1133 are configured to inhibit, limit, or prevent distal movement of the operative portion (e.g., valve) connected to the hub 1 175 when the distal anchors 1134 settle in the lumen wall.

FIGS. 12A-12C shows an example shape of a pinch-type anchoring mechanism having a plurality of elongated distal anchor struts used with a medical device inside a lumen. As shown in FIG. 12A, the pinch-type anchoring mechanism having a plurality of elongated distal anchor struts 1100 can comprise a pinch-type anchoring mechanism 1100 and an operative portion 1135. The operative portion 1135 can comprise a lung airway valve. The hub 1175 can connect the pinch-type anchoring mechanism 1100 to the operative portion 1 135.

As seen in FIGS. 12A to 12C, the elongated distal anchoring struts 1134 can transition from a straightened configuration and gradually return to a curved configuration. As seen in FIGS. 12A to 12C, the lumen 1110 can form a lumen wall pleat 1115 as the elongated distal anchoring struts 1134 changes shape. The lumen wall pleat 1115 can form between the elongated distal anchoring struts 1134 and opposing struts 1133. In some embodiments, the opposing struts 1133 include a bifurcated tip including a pad configured to limit an extent to which the opposing struts 1133 pierce the lumen 1100.

The elongated distal anchoring struts 1134 can comprise a memory-shape material. The elongated distal anchoring struts 1134 can change shape in response to electronic actuation, temperature changes, physical manipulation, dimensional changes resulting from releasing the device from a catheter, etc. For example, the elongated distal anchoring struts 1134 in a straightened form as shown in FIG. 12A can be activated by passing an electric current. The elongated distal anchoring struts 1134 can form a curved shape when activated as shown in FIG. 12C. The elongated distal anchoring struts 1134 in a curved form in FIG. 12C can be activated to straighten. For example, activating the elongated distal anchoring struts 1134 in FIG. 12C can straighten the elongated distal anchoring struts 1134 to form a straightened shape in FIG, 12A. In some embodiments, the struts 1133, 1134 are biased to their respective deployed configurations and are mechanically straightened (e.g., via a catheter and/or forceps) to fit within a catheter or working channel of an endoscope.

### Anchoring tips

Bodily movements (e.g., inhalation and/or exhalation, coughing, etc.) can cause a medical device used inside the lumen to move in different directions. Anchoring tips pointing in different directions can anchor a device to counteract such movement of the device. FIGS. 13A to 13C show different views an example shape of a bi-directional anchoring tip 1340. The bi-directional anchoring tip 1340 can be connected to a strut member 1330. The strut member 1330 can comprise pre-formed bends 1392A, 1392B. The strut member 1330 can be connected to an anchor hub 1375. In some embodiments, the anchor hub 1375 is connected to an operative portion (e.g., valve) of a medical device. The bi-directional anchoring tip 1340 can comprise a first anchoring tip 1335A and a second anchoring tip 1335B. In some embodiments, the first and/or second anchoring tips 1335A, 1335B extend from a fork body 1337.

The first anchoring tip 1335A and the second anchoring tip 1335B can extend from the fork body 1337. The fork body 1337 can connect the first anchoring tip 1335A and the second anchoring tip 1335B to the strut member 1330. In some embodiments, the anchoring tips 1335A, 1335B are formed from introducing a slit to a distal end of the strut member 1330 to split the distal end of the strut member 1330 into two separate portions (e.g., the first and second tips 1335A, 1335B). The first anchoring tip 1335A can extend generally in the same direction as the strut member 1330. For example, the first anchoring tip 1335A can extend in a radial direction from the hub 1375. The second anchoring tip 1335B can comprise a bend. The second anchoring tip I 335B can form an angle with the first anchoring tip 1335 A. For example, the second anchoring tip 1335B can bend at about 10 degrees to about 170 degrees from the fork body 1337. The first anchoring tip 1335A and the second anchoring tip 1335B can comprise different tip structures. For example, the first anchoring tip 1335A can comprise a sharp tip structure, such as the tip structure for the first anchoring tip 1335A shown in FIGS. 13A- 13C. The second anchoring tip 1335B can comprise a blunt tip structure as shown in FIGS. 13A- 13C.

The first anchoring tip 1335A can be configured to penetrate the lumen. The second anchoring tip 1335B can be configured to limit depth of penetration by the first anchoring tip 1335A. The second anchoring tip 1335B can point proximally, as shown in FIG. 13A. The second anchoring tip 1335B can be configured to exert radial outward force to the lumen wail. For example, the second anchoring tip 1335B can be configured to inhibit or prevent the movement of the device in a longitudinal direction. The proximal direction of the second anchoring tip 1335B can be used to obstruct movement of a medical device used with the bidirectional anchoring tip 1300 in a proximal direction inside a lumen.

The second anchoring tip 1335B can be located on the strut member 1330, away from the first anchoring tip 1335A. The second anchoring tip 1335B can comprise a sharp-edged bend. The first anchoring tip 1335A can form an angle with the fork body 1337.

### Membrane- frame struts

An endoluminal device having a shorter overall axial length can be useful. For example, an endoluminal device with shorter axial length can be used in a short lumen. The shorter axial length of the device can be used where the anchoring portion needs to be close to the operative portion, for example, to avoid anchoring a device on abraded patches of lumen wall. In some cases, use of a shorter device can be beneficial when the device is implanted adjacent an intersection of multiple branches of an airway or other body lumen.

FIGS. 14A to 14C show perspective views of an example endoluminal device 1400 having interposing strut members. The device 1400 can include a hub 1420. In some embodiments, the device 1400 includes an anchoring portion 1432 connected to the hub and an operative portion 1435 connected to the hub 1475. The anchoring portion 1432 can comprise a plurality of anchoring struts 1432. The plurality of anchoring struts 1432 can comprise anchoring tips 1440. The anchoring tips can comprise bi-directional tips 1335A, 1335B (shown in FIGS. 13A and 13B). The operative portion 1425 can include membrane struts 1435 and a membrane 1463 attached to the membrane struts 1435. The operative portion 1425 (e.g., the membrane struts 1435) can be connected to and extend proximally from the hub 1475. The device 1400 can include a center rod 1460 connected to the hub 1475 and positioned between the membrane struts 1435 and a forceps knob 1462 on a proximal end of the center rod 1460.

The anchoring shifts 1432 can comprise a bent shape having anchoring tips 1440 pointing in a radially-outward direction, as shown in FIGS. 14A to 14C. In some embodiments, the anchor struts 1432 include a pad or other structure adjacent the tips 1440 to limit an extent to which the tips 1440 penetrate an airway wall. As shown in FIG. 14B, the anchoring struts 1432 can be interposed with the frame struts 1435 around a circumference of the device 1400, such that the anchoring tips 1440 can be interposed with the frame strut tips 1436 when viewed in a transverse direction. In some cases, the number of anchoring tips 1440 and anchor struts 1432 matches the number of struts 1435. Interposing of the struts 1435, 1432 can be used, for example, to minimize the total diameter of the endoluminal device 1400 when compressed within a delivery catheter.

The anchoring struts 1432 can extend outward from the hub 1475 and bend in a proximal direction as shown in FIGS, 14A-14C. In some embodiments, the anchor struts 1432 are configured to exert a radially-outward force on a lumen in which the device 1400 is deployed. The operative portion 1435 can be disposed proximally to the anchoring portion 1432 when placed inside the lumen. When placed inside the lumen, the frame strut tips 1436 of the operative portion 1435 and the anchoring tips 1440 can be placed apart at a distance D4. The distance D4 between the anchoring tips 1440 and the frame strut tips 1436 of the operative portion 1425 can be large enough to reduce the risk of contact between the tips 1440 and the membrane 1463. The distance D4 can be about .02mm to about 5mm. The distance D4 can be about .05mm to about 4mm. The distance D4 can be about .1mm to about .3mm. The distance D4 can be about .15mm to about 2mm. The distance D4 can be about .2mm to about 1mm.

Positioning the anchoring tips 1440 near the frame strut tips 1436 can reduce movement of the operative portion 1425 when the anchor tips 1440 settle in the walls of the lumen in which the device 1400 is deployed. For example, the anchoring struts 1432 can be configured to limit movement of the operative portion 1435 to be less than about .2mm. The anchoring struts 1432 can be configured to limit movement of the operative portion 1435 to be less than about .15mm. The anchoring struts 1432 can be configured to limit movement of the operative portion 1435 to be less than about .05mm. The anchoring struts 1432 can be configured to limit movement of the operative portion 1435 to be less than about .02mm. The anchoring struts 1432 can be configured to limit movement of the operative portion 1435 to be less than about .01 mm.

The anchoring struts 1432 can comprise a plurality of pre -formed bends. In some cases, anchoring struts 1432 can have a curved shape without identifiable bends. The anchoring struts 1432 can have uniform thickness along a length of the struts 1432. In some embodiments, some portion of the struts 1432 is thicker than another portion of the struts 1432. The thick portions can be configured to have less flexibility than thinner portions. For example, the thinner portions can be configured to allow greater bending than the thicker portions.

The endo luminal device 1400 can be transported using a delivery catheter. For example, the interposing anchoring struts 1432 can be folded together with the frame struts 1435 while interposing the frame struts 1435 to fit inside the catheter. The endo luminal device 1400 can be deployed by being pushed out of the catheter. In some embodiments, the endoluminal device 1400 is deployed by withdrawing the catheter from the endoluminal device 1400 when the device 1400 is positioned at the deployment site. The endoluminal device 1400 can be pushed out such that the hub 1475, the anchoring tip 1440, and the membrane strut tips 1432 can leave the catheter sequentially. Once the anchoring tip 1440 leaves the catheter, the anchoring tip 1440 can latch onto the surface of the lumen 1410. A user can pull the catheter away from the frame struts 1435 once the anchoring tip 1440 is latched onto the lumen 1410.

In some embodiments, the anchoring struts 1432 having a pre-formed bent shape can be straightened to extend at a distal direction from the hub 1475 to fit inside and be transported across the catheter. The anchoring struts 1432 can comprise a different number of struts as the number of the frame struts 1435 A.

### Connecting member

Anchoring struts can be coupled to an operative portion by an extendable and/or flexible connecting member. FIGS. 15A and 15B show an example of an endoluminal device 1500 having an extendable connecting member 1560. The endoluminal device 1500 can comprise an operative portion 1535, a connecting member 1560 connected to the operative portion 1535, and an anchoring portion 1530 connected to the connecting member 1560. The anchoring portion 1530 can comprise a distal hub 1575B, anchoring struts 1532 connected to and extending from the distal hub 1575B, and anchoring tips 1540. The anchoring tips 1540 can comprise a first tip 1536A and a second tip 1536B. The second tips 1536B can be configured to limit a depth to which the first tips 1536A pierce into the walls of a lumen in which the endoluminal device 1500 is installed. The operative portion 1535 can comprise a proximal hub 1575 A, a plurality of frame struts 1535A connected to and extending from the proximal hub 1575A, and a membrane 1535B connected to the frame struts 1535 A.

The anchoring portion 1530 can be disposed distally from the operative portion 1535. The anchoring struts 1530 can extend radially from the distal hub 1575B and generally in a distal direction. The frame struts 1535A can comprise a bent surface 938 (as shown in Fig. 9). The bent surface 937 can be configured to minimize trauma to the lumen 1510. The frame struts 1535A can extend proximally from the proximal hub 1575A. The connecting member 1560 can connect the distal hub 1575B and the proximal hub 1575 A. The connecting member 1560 can connect the anchoring portion 1530 and the operative portion 1535. The connecting member 1560 can be extendable. For example, the connecting member 1560 can be a helical spring as shown in FIGS. ISA and I5B.

In a first configuration shown in FIG. 15A, the connecting member 1560 can contract to bring the operative portion 1535 and the anchoring portion 1530 close together. In a second configuration shown in FIG. 15B, the connecting member 1560 can extend, and the operative portion 1535 and the anchoring portion 1530 can be spaced farther apart from each other. The distance between the proximal hub 1575 A and the distal hub 1575B can be greater in the second configuration compared to the first configuration.

The connecting member 1560 can be extended and/or contracted in response to change in lumen dimension. The connecting member 1560 can be sufficiently elastic to allow axial movement of the anchoring portion 1530 without displacing the operative portion 1535. In some embodiments, the anchoring portion 1530 can move away from the operative portion 1535, as the anchor struts 1532 and lumen walls 1515 expand outward. In some cases, movement of the anchoring portion 1530 away from the operative portion 1535 can be accomplished with little or no movement of the operative portion 1535 toward the anchoring portion 1530.

The connecting member 1560 can comprise a tensile strength sufficient to hold the operative portion 1535 in place, regardless of change in lumen 1510 dimension. For example, the connecting member 1560 can be configured to counteract the contraction of the lumen 1510, such that while the connecting mechanism 1560 initially extends during lumen contraction, the strength of the connecting mechanism 1560 can spring back to its original length and re-expand the lumen 1510. The spring portion 1560 can comprise an elastic structure, such as an elastomer. For example, the spring portion 1560 can comprise synthetic rubber, silicone, etc.

### Connecting member in bifurcating lumen

The endoluminal device 1500 can be used in bifurcating lumen, such as bifurcating airways. The extendable connecting member 1560 can be used in airways having small diameters. FIGS. 16A and I6B show an example of an endoluminal device 1500 having an extendable connecting member used in a bifurcating lumen 1610. The bifurcating lumen 1610 can comprise a first lumen 1615A and a second lumen 1615B. The first and second lumens 1615A, 1615B can comprise different dimensions. For example, the first lumen 1615A can comprise a smaller diameter than the diameter of the second lumen 1615B. The first and second lumens 1615 A, 1615B can expand or contract individually or together.

FIG. 16A shows the endoluminal device 1500 disposed mostly in the first lumen 1615A. The anchoring portion 1530 can be disposed distally in the first lumen 1615A and the operative portion 1535 can be disposed on or near an entrance of the first lumen 1615A. The operative portion 1535 can contact the first lumen 1615A at an expanded contact diameter 159GB. The contact diameter 1590B can comprise a diameter of the first lumen 1615A in which portions of an outer surface of the operative portion comes in contact with the lumen 1615 A.

FIG 16B shows the first lumen 1615A in a contracted state. In a contracted state, the first lumen 1615A can exert a force on the operative portion 1535 and portions of the operative portion 1535 can be disposed outside of the first lumen 1615A. For example, the operative portion 1535 can contact the first lumen 1615A at a contracted contact diameter 1590A. The contracted contact diameter 1590A can be closer to the proximal hub 1575A than the expanded contact diameter 1590B. The contracted contact diameter 1590 A can be smaller than the expanded contact diameter 1590B.

The connecting portion 1560 can elastically extend to allow the operative portion 1535 to move proximally while the first lumen 1615A contracts. The connecting portion 1560 can pull the operative portion 1535 distally as the first lumen 1615A expands. The elastic extension and the pulling of the connecting portion can apply a sufficient counter force to keep the endoluminal device 1500 seated inside the airway, without causing the anchoring mechanism to be removed from its position. The distance which the connecting portion 1560 can extend can be determined by the angle of the cone of the valve portion and the diameter of the airway wall that the operative portion 1535 covers.

The struts 1535A in the operative portion 1535 can rest against an opening of the first lumen 1615A and into a larger lumen 1605 to counteract pulling from the anchoring portion 1530 as shown in FIG. 16A and 16B. The anchoring portion 1530 can remain in the same location in the first lumen 1615A during expansion and contraction. In some cases, the anchoring portion 1530 moves distally within the first lumen 1615A during settling of the anchoring portion 1530. For example, in the expanded first lumen 1615A in FIG. 16A, the connecting member 1560 can be at a first length D5. In the contracted first lumen 1615A of shown in FIG. 16B, the connecting member 1560 can be at a second length D6. The first length D5 can be shorter than the second length D6. The operative portion 1535 can move further out to the larger lumen 1605 in a proximal direction as the first lumen 1615A contracts. The distance between the proximal hub 1575A and the distal hub 1575B can increase during the contraction of the lumen 1615A. The distance between the proximal hub 1575A and the distal hub 1575B can decrease during the expansion of the lumen 1615A. The elastic force generated by the connecting portion 1560 can be tuned to hold the operative 1535 portion against the opening.

The operative portion 1535, while suited for a larger airway, can be used in a smaller airway with an opening to a larger airway. As shown in FIGS. 16A and 16B, the operative portion 1535 can be nested in the proximal end of the first lumen 1615A. For example, the first lumen 1615A can comprise a contact diameter 159GA smaller than an outermost diameter of the operative portion 1535. The outermost diameter of the operative portion 1535 can be the largest diameter of the operative portion 1535. A portion of the operative portion 1535 can be located inside the first lumen 1615A while the remaining portion of the operative portion 1535 can extend outside of the entrance of the first lumen 1615A. The shape and size of the operative portion 1535 can be configured to ensure airflow through second lumen 1615B.

## Claims

1. A mechanism (400) for anchoring endoluminal devices to a lumen, the mechanism comprising:
a hub member (420) configured to be located at a predetermined location inside the lumen; and
a plurality of anchoring struts (430), the plurality of anchoring struts comprising:
a spring portion attached to the hub member and configured to extend or contract during expansion and contraction of the lumen; and
an anchoring portion (440) connecting the spring portion to the lumen, the anchoring portion configured to penetrate a portion of the lumen;
wherein the hub member is positioned on a plane with the anchoring portions of the plurality of anchoring struts, and
wherein each of the spring portion of the plurality of anchoring struts comprise bent strut members configured to straighten during expansion of the lumen and bend during contraction of the lumen,
**characterized in that** each of the bent strut members comprise undulating strut members having a substantially straight shape when viewed in a transverse direction.

2. The mechanism of claim 1, wherein the anchoring portion comprises a bidirectional anchoring tip (640) comprising a first tip (640A) pointed to a first direction and a second tip (640B) pointed to a second direction at an angle from the first direction, wherein at least a portion of the first tip penetrates the lumen in a transverse direction to the lumen.

3. The mechanism of claim 1, wherein each of the plurality of anchoring struts comprise equal lengths.

4. The mechanism of claim 1, wherein each of the spring portions of the plurality of anchoring struts extends radially outward from the hub member.

5. The mechanism of claim 1, wherein the bent strut members comprise one or more thin portions (433B) and one or more thick portions (433A) thicker than the thin portions.

6. The mechanism of claim 1, wherein the hub member comprises a first hub and a second hub (722) collinear and connected to the first hub, wherein the plurality of anchoring struts are connected to the first hub.

## Patentansprüche

1. Ein Mechanismus (400) zur Verankerung von endoluminalen Vorrichtungen an einem Lumen, wobei der Mechanismus umfasst:
ein Nabenelement (420), das so eingerichtet ist, dass es an einer vorbestimmten Stelle innerhalb des Lumens angeordnet wird; und
eine Vielzahl von Verankerungsstreben (430), wobei die Vielzahl von Verankerungsstreben umfasst:
einen Federabschnitt, der an dem Nabenelement angebracht und eingerichtet ist, sich während der Expansion und Kontraktion des Lumens auszudehnen oder zusammenzuziehen; und
einen Verankerungsabschnitt (440), der den Federabschnitt mit dem Lumen verbindet, wobei der Verankerungsabschnitt eingerichtet ist, einen Abschnitt des Lumens zu durchdringen;
wobei das Nabenelement in einer Ebene mit den Verankerungsabschnitten der Vielzahl von Verankerungsstreben angeordnet ist, und
wobei jeder der Federabschnitte der Vielzahl von Verankerungsstreben gebogene Strebenelemente umfasst, die eingerichtet sind, sich während der Expansion des Lumens zu strecken und während der Kontraktion des Lumens zu biegen,
**dadurch gekennzeichnet, dass** jedes der gebogenen Strebenelemente wellenförmige Strebenelemente umfasst, die in einer Querrichtung gesehen eine im Wesentlichen gerade Form aufweisen.

2. Der Mechanismus nach Anspruch 1, wobei der Verankerungsabschnitt eine bidirektionale Verankerungsspitze (640) umfasst, die eine erste Spitze (640A) umfasst, die in eine erste Richtung gerichtet ist, und eine zweite Spitze (640B), die in eine zweite Richtung in einem Winkel zur ersten Richtung gerichtet ist, wobei zumindest ein Teil der ersten Spitze das Lumen in einer Querrichtung zum Lumen durchdringt.

3. Der Mechanismus nach Anspruch 1, wobei jeder der mehreren Verankerungsstreben gleiche Längen aufweist.

4. Der Mechanismus nach Anspruch 1, wobei sich jeder der Federabschnitte der Vielzahl von Verankerungsstreben radial von dem Nabenelement nach außen erstreckt.

5. Der Mechanismus nach Anspruch 1, wobei die gebogenen Strebenelemente einen oder mehrere dünne Abschnitte (433B) und einen oder mehrere dicke Abschnitte (433A) aufweisen, die dicker als die dünnen Abschnitte sind.

6. Der Mechanismus nach Anspruch 1, wobei das Nabenelement eine erste Nabe und eine zweite Nabe (722) umfasst, die kollinear mit der ersten Nabe und mit dieser verbunden ist, wobei die Vielzahl der Verankerungsstreben mit der ersten Nabe verbunden ist.

## Revendications

1. Mécanisme (400) pour ancrer des dispositifs endoluminaux à une lumière, le mécanisme comprenant:
un élément de moyeu (420) configuré pour être situé à un emplacement prédéterminé à l'intérieur de la lumière; et
une pluralité d'entretoises d'ancrage (430), la pluralité d'entretoises d'ancrage comprenant:
une partie ressort fixée à l'élément moyeu et configurée pour s'étendre ou se contracter pendant l'expansion et la contraction de la lumière; et
une partie d'ancrage (440) reliant la partie ressort à la lumière, la partie d'ancrage étant configurée pour pénétrer dans une partie de la lumière;
dans lequel l'élément de moyeu est positionné sur un plan avec les parties d'ancrage de la pluralité d'entretoises d'ancrage, et
dans lequel chacune des parties de ressort de la pluralité d'entretoises d'ancrage comprend des éléments-entretoises courbés configurés pour se redresser pendant l'expansion de la lumière et se courber pendant la contraction de la lumière,
**caractérisé en ce que** chacun des éléments-entretoises courbés comprend des éléments-entretoises ondulés ayant une forme essentiellement droite lorsqu'ils sont vus dans une direction transversale.

2. Le mécanisme de la revendication 1, dans lequel la partie d'ancrage comprend une pointe d'ancrage bidirectionnelle (640) comprenant une première pointe (640A) dirigée vers une première direction et une seconde pointe (640B) dirigée vers une seconde direction à un angle par rapport à la première direction, dans lequel au moins une partie de la première pointe pénètre dans la lumière dans une direction transversale à la lumière.

3. Le mécanisme de la revendication 1, dans lequel chacune de la pluralité d'entretoises d'ancrage est constituée de longueurs égales.

4. Le mécanisme de la revendication 1, dans lequel chacune des parties de ressort de la pluralité d'entretoises d'ancrage s'étend radialement vers l'extérieur à partir de l'élément de moyeu.

5. Le mécanisme de la revendication 1, dans lequel les éléments-entretoises courbés comprennent une ou plusieurs parties fines (433B) et une ou plusieurs parties épaisses (433A) plus épaisses que les parties fines.

6. Le mécanisme de la revendication 1, dans lequel l'élément de moyeu comprend un premier moyeu et un second moyeu (722) colinéaire au premier moyeu et connecté à celui-ci, dans lequel la pluralité d'entretoises d'ancrage sont connectées au premier moyeu.
